# EUROPEAN PATENT SPECIFICATION

(11) **EP 4 213 833 B1**
(45) Date of publication and mention of the grant of the patent: **18.09.2024**
(21) Application number: 21787500.4
(22) Date of filing: 18.09.2021
(51) Int. Cl.: A61K 31/35, A61K 45/06, A61P 35/00, A23L 11/00, A23L 33/00

(54) **KOJIC ACID DERIVATIVE AS SELECTIVE INHIBITOR OF MITOSIS IN COLORECTAL AND GLIOBLASTOMA CANCER CELLS**
KOJISÄUREDERIVAT ALS SELEKTIVER MITOSEHEMMER IN KOLOREKTALEN UND GLIOBLASTOMA-KREBSZELLEN
DÉRIVÉ D'ACIDE KOJIQUE COMME INHIBITEUR SÉLECTIF DE LA MITOSE DANS LES CELLULES DE CANCER COLORECTAL ET DE GLIOBLASTOME

(30) Priority: 18.09.2020 IT 202000022066
(43) Date of publication of application: 26.07.2023
(73) Proprietor: Università Degli Studi di Cagliari, 09124 Cagliari (IT)
(72) Inventor: LACHOWICZ, Joanna lzabela, 09124 Cagliari (IT); PICHIRI, Giuseppina, 09124 Cagliari (IT); PIRAS, Monica, 09124 Cagliari (IT); PILUDU, Marco, 09124 Cagliari (IT); ORRÙ, Germano, 09124 Cagliari (IT); CONI, Pierpaolo, 09124 Cagliari (IT); CONGIU, Terenzio, 09124 Cagliari (IT)
(74) Representative: Primiceri, Maria Vittoria
(86) International application number: PCT/IB2021/058522
(87) International publication number: WO 2022/058970

(56) References cited:
- CHO D H ET AL: "Composition useful for treating cancer e.g. astrocytoma by suppressing cell proliferation, and enhancing necrosis, comprises kojic acid derivative", WPI / 2017 CLARIVATE ANALYTICS,, vol. 2011, no. 58, 25 May 2011 (2011-05-25), XP002802977
- DATABASE WPI Week 201158, Derwent World Patents Index; AN 2011-G97696, XP002802977
- NURCHI VALERIA M ET AL: "Equilibrium studies of new bis-hydroxypyrone derivatives with Fe3+, Al3+, Cu2+and Zn2+", JOURNAL OF INORGANIC BIOCHEMISTRY, ELSEVIER INC, US, vol. 189, 17 September 2018 (2018-09-17), pages 103 - 114, XP085518637, ISSN: 0162-0134, DOI: 10.1016/J.JINORGBIO.2018.09.013

## Description

### Technical field

The present invention relates to to the production of kojic acid derivatives which are able to selectively block the cell cycle of cancer cells, in particular colorectal and glioblastoma cancer cells. The same can be used in combination with various pharmaceutical preparations to be used in the treatment of specific clinical cases and in the veterinary field.

### Prior art

Until now, most of the anticancer therapies have focused on the inhibition of cell proliferation without however being able to have a specific selectivity towards particular tumor cells, these treatments affect not only the pathological cells, but also the normal ones. For this reason, several therapeutic strategies have been developed capable of selectively acting on the tumor and not on normal cells [*"*Mitosis inhibitors in anticancer therapy: When blocking the exit becomes a solution" Cancer Letters 440-441 (2019) 64-81*].*

One of these involves the use of humanized monoclonal antibodies. In fact, in 2015 the FDA approved one of these treatments in the clinical practice of the orphan treatment of colorectal cancer based on the compound Panitumumab, (Vectibix, Amgen) (*https:*//*www.healio.com*/*hematology-oncology*/*gastrointestina-cancer*/*news*/*print*/*hemonc-today*/*%7B630b0124-b087-4b14-8c99-a1fbeb52f06a%7D*/*fda-approves-new-colorectal-cancer-drug,* FDA approves new colorectal cancer drug), a humanized monoclonal antibody produced in animals from laboratory and subsequently modified to make it compatible with human immunoglobulins. This antibody selectively recognizes the mutated Epidermal Growth Factor receptor or (the EGFR transmembrane protein) which is frequently found altered in this type of tumors and which is at the origin of the uncontrolled proliferative stimulus of these neoplastic cells (and which is now available also in our oncology departments). EGFR binds to epidermal growth factor by stimulating cell proliferation (E. Horta, et al. Clinical Neurology and Neurosurgery 188 (2020) 105566).

However, humanized EGFR, like other classes of antibodies used in cancer pharmacotherapy, has neurotoxic side effects (headache with a frequency of 25% of cases; neuropathy, 16%; sleep disturbances, 15%). Wider and more severe symptoms (e.g., brain edema) have been observed in individuals with pre-existing brain injuries [E. Horta, et al. Clinical Neurology and Neurosurgery 188 (2020) 105566]. Furthermore, only 60-70% of all colorectal cancers respond to treatment and test positive for EGFR and can therefore be treated with Panitumumab-based therapies, highlighting the need for alternative therapies.

Another drawback of these biological therapies is the high cost of the drug. The use of antibodies in cancer therapy is a huge step forward, but the high cost of such therapies makes them accessible only in some for the most economically developed countries. For these reasons it is necessary to identify subjects who potentially have the molecular characteristics that make them sensitive to treatment and it is therefore envisaged to carry out, before treatment, a series of molecular tests that indicate the targeted use of the drug to the oncologist.

In patent document KR101126957 the kojic acid derivatives in the treatment of tumors are described; however, the compounds described were not selective towards specific cell lines. Furthermore, the synthesis of the compounds patented in document KR101126957 is'multi-step' and requires a minimum of three syntheses (Step-by-Step) in order to obtain the final product.

Our invention responds to the need to have a therapeutic alternative in cancer, in particular colorectal and glioblastoma cancers, using kojic acid derivatives.

### Summary of the invention

The rationale for this project is based on our data performed *in vitro* on cell lines derived from different tumor types. The data obtained from these experiments indicate that only cells obtained from colorectal and glioblastoma tumors are sensitive to the inhibitory effect exerted by the L1 compound on cell proliferation. Cell lines from other tumors or so-called normal cells show no obvious sensitivity to treatment. Furthermore, the synthesis of compound L1 is a simple, fast and inexpensive one-step synthesis. Finally, it should not be overlooked that this type of treatment can be inexpensive, feasible in all hospital departments and potentially usable in combination with other antineoplastic drugs, increasing the possibilities of therapeutic alternatives as required by our current knowledge. Our experimental results indicate that compound L1 is not only selective but, at least *in vitro,* does not exhibit toxic side effects.

Therefore, the main purpose of this invention is to propose the development of a compound with pharmacological activity that can be used in medical oncology and possibly in veterinary medicine.

The compounds indicated in the following description are derivatives of kojic acid, among these the compound L1 can selectively block the cells of some tumors in the mitotic phase of their cell cycle. In particular, some preliminary results we obtained *in vitro* on different cell lines suggest a certain sensitivity to these molecules by colorectal and glioblastoma cancer cells. It would therefore be possible to use compound L1 in primary tumors, metastases and relapses, as a single treatment or in combination with other antineoplastic drugs.

Another object of the invention are the pharmaceutical compositions comprising the L1 derivative of kojic acid in combination with one or more pharmaceutically acceptable carriers, excipients or diluents.

Further objects and advantages obtainable from the use of the compound L1 will be indicated by the detailed description of the invention.

### Brief Description of Figures

**Figure 1****.** 293T, RAW, T98G and CaCo2 cell samples stained with hematoxylin and eosin. Panels A, B, G, L show the control cells; panels B, E, H, M the cells treated with Colcemid (5.41 mM); panels C, F, I, N the cells treated with L1 (0.74 mM).
**Figure 2****.** Cell cultures of Caco-2 treated with L1 (0.74 mM). The analysis with optical microscopy (panel A) highlights the presence of scattered rounded cells, characterized by the absence of the nuclear membrane or with sporadic fragmented nuclei (highlighted with the arrow). The asterisk indicates the presence of cells blocked in the mitotic phase. Size bar = 25mm. The higher resolution of transmission electron microscopy (panel B) highlights the ultrastructural details of the fragmented nuclei of L1-treated Caco-2 cells, (arrow). Size bar = 2mm. Mv = microvilli.
**Figure 3****.** The graphs show the curve over time of the number of Caco2 cells treated with L1 (gray color; conc. L1 0.74 mM).) And those of the corresponding control (black color, untreated cells) obtained with the system of automatic cell count LunaFL.
**Figure 4****.** Curve over time of the viability percentages obtained with the LunaFL automatic cell counting system of Caco2 cells treated with L1 (gray color; conc. L1 0.74 mM) and those of the corresponding control (black color, cells not treated).
**Figure 5****.** 293T and CaCo2 cell lines photographed under a light microscope. Panels A, D show the control cells; panels B, E cells treated with Colcemid (5.41 mM); panels C, F cells treated with L1 (0.74 mM). After 24 hours the cells were treated with Trypan Blue for viability studies.
**Figure 6****.** The expression results of oxidative stress genes in 293T and Caco-2 cells treated 24 hours with L1 (0.74 mM) compared to control samples in normal growth medium. BAX (Up / downregulated): promotes apoptosis, BCL2 (Up / downregulated): regulates the permeabilization of the mitochondrial outer membrane (MOMP); role for reduced apoptosis; OH-1 (Upregulated): in response to oxidative stress hypoxia, heavy metals, cytokines; PRDX (Upregulated): oxidative stress gene. (1B = control sample; 2B = treated sample L1).

### Detailed description

The present invention is defined by the claims. Any subject-matter falling outside the scope of the claims is provided for information only. The present invention relates to the synthesis of a compound that can be used as a drug in the treatment of tumors in general and of the glia and intestine, in particular. More specifically, the compound of the invention is proposed as a drug for the specific treatment of glioblastoma and colorectal cancer, based on preliminary results obtained in vitro on colon adenocarcinoma cell lines (CaCo2) where the compound induces selective block of mitoses.

The proposed invention also describes the synthesis of a compound which can be used as a drug for the specific treatment of the tumor, in particular of the colorectal, whose function is the selective block of mitosis of tumor cells only.

The invention therefore refers to a possible use of a kojic acid derivative, called L1 and consisting of two units of kojic acid and ethylenediamine (linker). According to laboratory studies conducted on different types of cells and comparative studies with similar molecules (L2, L3, L4), it has been shown in vitro that the L1 compound can inhibit the mitosis process (cell division process) by acting selectively on cells colorectal and glioblastoma cancers.

In these experiments, four similar compounds (Scheme 1) based on kojic acid were synthesized and tested.

These compounds are obtained through thesynthesis *one-step* of kojic acid (IUPAC name: 5-hydroxy-2-(hydroxymethyl)-4*H*-pyran-4-one or even 5-hydroxy-2-(hydroxymethyl)-4-pyrone, 2-hydroxymethyl-5-hydroxy-y-pyrone CAS: 501-30-4) and ethylenediamine (CAS: 107-15-3). This synthesis has the advantage of being quick and cheap (procedure described in the scientific article *Equilibrium studies of new bis hydroxypyrone derivatives with Fe3+, Al3*+*, Cu2+ and Zn2+* Journal of lnorganic Biochemistry 189 (2018) 103-114).

The four compounds described above are known in the scientific literature as chelators of metals (iron, copper, zinc and aluminum) and of phosphate anions (eg monophosphate, pyrophosphate, polyphosphate, ATP, ADP, AMP).

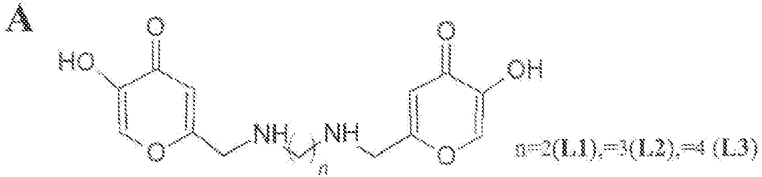

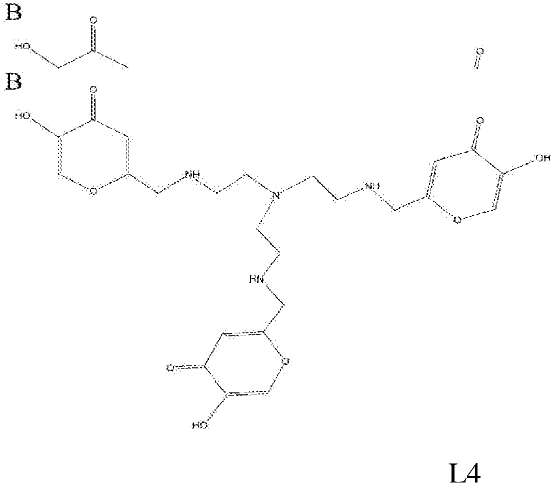

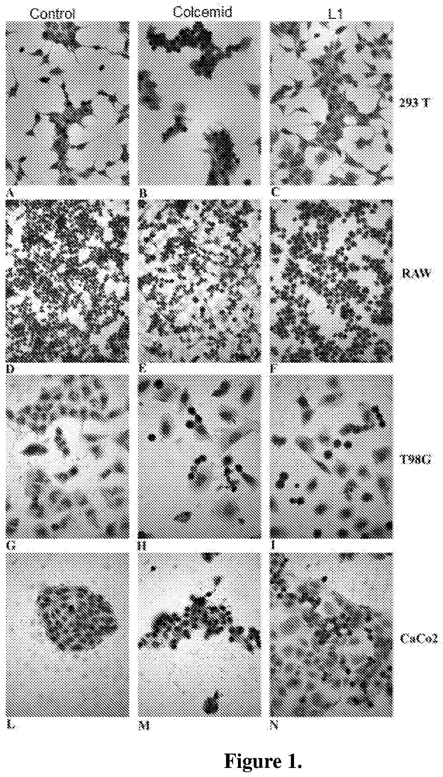

The results obtained indicate that only the L1 molecule and not those (L2, L3 and L4) showed antiproliferative activity (through the inhibition of mitosis) in tumor cells: CaCo2 and T98G. The same L1 molecule showed no effect in other non-tumor cell lines such as those derived from embryonic kidney cells (293 cells) and human macrophages (J774 cells) (Figure 1).

For each of the compounds, different culture media were used in which tumor and non-tumor cell lines were grown. After 24 hours of culture, only Caco2 (colon adenocarcinoma) and L1-treated T98G (human glioblastoma) cells showed morphological changes characteristic of mitotic-blocked cells. When the same experiment was repeated by replacing the L1 molecule with *colcemide* (known antimitotic drug, Bulletin der Schweizerischen Akademie der Medizinischen Wissenschaften; Volume 12, Issue 2, June 1956, Pages 163-173; "Differentiated inhibition of mitosis by colcemid (desacetylmethylcolchicine) injection at various times of the day". Journal of Ultrasructure Research; Volume 19, Issue 1-2, July 1967, Pages 1-18; "The effects of Colcemid inhibition and reversal on the fine structure of the mitotic apparatus of Chinese hamster cells in vitro" Cancer Research; Volume 54, Issue 18, September 1994, Pages 5011-5015; "Cell Cycle Arrest by Colcemid Differs in Human Normal and Tumor Cells") all cell lines studied showed the morphological changes characteristic of blocked cells in mitosis. Therefore, only the Caco2 and T98G cells treated with L1 showed a morphology indicative of a block in mitosis, identical to that induced by the administration of colcemide.

Ultrastructural analysis using SEM and TEM microscopy confirmed that compound L1 acts specifically on the cell division (mitosis) process of Caco2 cells.

We experimentally established the minimum inhibitory concentration and the minimum time of treatment with L1. After 4.5 hours of treatment, half of the Caco2 cells were blocked in mitosis, the block of all treated cells was achieved after 24 hours of treatment. This block in mitosis resulted in a reduction in the number of cells which was on average 4 times lower than in the control sample (same cell line cultured without L1). The minimum concentration of the binder, which is able to induce evident morphological changes after 24 hours of treatment, was found to be 0.069 mM. As the ligand concentration increases, the number of cells modified by the ligand increases and, in the solution containing the ligand at a concentration of 2.22 mM, all the cells have a modified morphology.

It has been noted that the process of mitosis inhibition is reversible if the treatment with L1 ligand is stopped after 24 hours (the culture medium with L1 is replaced with one without this compound). If, on the other hand, the treatment with L1 is continued for up to 72 hours, the inhibition of mitosis is irreversible and the Caco2 cells begin to die from apoptosis.

These preliminary data support the possibility that compound L1 could be used as a drug for humans and possibly for veterinary use.

Furthermore, the present invention provides an antitumor pharmaceutical composition for the treatment of cancer, in particular of the colorectal cancer and related relapses and metastases. The composition comprises as an active ingredient effective doses of the kojic acid-derived molecule L1 or a pharmaceutically acceptable salt thereof, in which the composition of the present invention is a drug for preventing and treating tumor symptoms in humans, including the elderly and children.

Furthermore, the composition according to the present invention can contain a pharmaceutically effective amount of the compound L1 derived from kojic acid in addition to one or more pharmaceutically acceptable carriers, excipients or diluents and can also be formulated as a pediatric formulation.

The pharmaceutically effective amount mentioned above refers to an amount sufficient to prevent, improve and cure the tumor and according to our preliminary data it could correspond to about 0.5 and 5 mg/day/kg of body weight. This dose must obviously be determined experimentally on the basis of the degree of disease symptoms, the patient's age, weight, state of health, sex, route of administration and period of treatment.

Examples of carriers and excipients could be lactose, dextrose, sucrose, sorbitol, mannitol, xylitol, erythritol, maltitol, starch, acacia gum, alginate, gelatin, calcium phosphate, calcium silicate, cellulose, methylcellulose, polyvinylpyrrolidone, water, talc, magnesium stearate and mineral oil.

Fillers, anticoagulants, lubricants, wetting agents, fragrances, emulsifiers and preservatives could be further added to the compositions.

Treatment could involve rapid, prolonged or delayed release of the active ingredient.

Since the L1 molecule selectively blocks the neoplastic cells in mitosis, this treatment could in this way synchronize or select neoplastic clones more sensitive to other chemotherapeutics. It is therefore also possible to propose a possible'mixed' anticancer therapy by combining the formulation of L1 with the other known anti-tumor drugs (eg cis-platinum and its derivatives: nedaplatin, carboplatin, picolplatin, sartaplatin, lobaplatin, oxaliplatin, carboplatin).

The pharmaceutical composition could be provided in the form of powders, granules, tablets, emulsions, syrups, aerosols, soft or hard gelatin capsules or sterile injectable solutions, and could therefore be administered orally, transdermally, subcutaneously, intravenously or intramuscularly, and the dosage of the active ingredient can vary according to the route of administration, the age of the patient, the sex, the weight and the severity of the patient.

Furthermore, the compositions can be administered in combination with a known compound having a preventive, ameliorating or treating effect of tumor symptoms (Pembrolizumab, Aflibercept, Bevacizumab, Cetuximab, Levoleucovorin, Nivolumab, Panitumumab, Tipiracil, Trifluridine, Vandetanib, 7-ethyl -10-hydroxycamptothecin, Fluorouracil, Cyanocobalamin, Capecitabine, CT-011, Buparlisib, Bevacizumab, Benzalkonium, Abemaciclib, Nilotinib, Capecitabine, Mitomycin, Ramucirumab, Regorafenib).

Compound L1 and related pharmaceutical compositions are not expected to cause clinically severe allergic reactions such as gastrointestinal disturbances, dizziness or similar reactions when administered to humans.

Compared to other known solutions for tumor therapy, compound L1 can be used directly without particular drug approaches, formulations, technologies or delivery systems. Furthermore, the compound of the invention has a high water solubility (> 0.6M) [Journal of Inorganic Biochemistry Volume 189, December 2018, Pages 103-114; Equilibrium studies of new bis-hydroxypyrone derivatives with Fe3+, Al3+, Cu2+ and Zn2+] and can therefore also be used in oral treatment (significantly easier and cheaper treatment than intravenous infusion therapy which requires hospitalization of the treated patient).

The compound L1 of the present invention can be added to foods or food products aimed at preventing or improving the tumor, and therefore the composition of the invention can be used as a composition for functional foods with anticancer effects. The composition for functional foods with antitumor effect of the present invention can be easily used as an effective food in the prevention and improvement of tumor symptoms, for example as a main raw material, as a food additive, or as a functional food or drink. The term "food" in this case refers to a natural product or a processed product that contains one or more nutrients and preferably indicates a formulation that can be directly consumed as a food, food additive, functional food and beverage, including infant formula and baby food.

Hereinafter, the present invention will be described in detail by examples. However, the following examples are provided for the sole purpose of illustrating the invention and are in no way to be considered as limiting its scope.

### EXAMPLES

### Example 1. Synthesis of compounds L1-L4

### Synthesis of compound L1

2,2'- ethane-1,2-diylbis (iminomethanediyl)]bis(5-hydroxy-4H-pyran-4-one) (L1). 1.0 g (7 mmol) of KA (Kojic Acid) was dissolved in 20 mL of ethanol (96%) and 230 µl (3.5 mmol) of ethylenediamine was added. After stirring the mixture for 1 hour at room temperature, the precipitate was filtered and washed with ethyl acetate. The identity and purity of the compound were ascertained by NMR analysis. Quantum yield 98%, melting point 138°C, ¹H NMR (500 Hz, D₂O), δ (ppm): 7.80 (s, 1H, 6-HPy), 6.42 (s, 1H, 3-HPy) , 4.39 (s, 2H, CH₂-Py), 2.95 (s, 2H, CH₂).

### Synthesis of the compound L2

2,2'-[propane-1,3-diylbis (iminomethanediyl)]bis(5-hydroxy-4H-pyran-4-one) (L2). 1.0 g (7 mmol) of KA was dissolved in 20 mL of ethanol (96%) and 290 µl (3.5 mmol) of propane-1,3-diamine was added. After stirring the mixture for 1 hour at room temperature, the precipitate was filtered and washed with ethyl acetate. The identity and purity of the compound were ascertained by NMR analysis. Quantum yield 91%, Melting point 145°C, ¹H NMR (500 Hz, D₂O), δ (ppm): 7.71 (s, 1H, 6-HPy), 6.38 (s, 1H, 3-HPy) , 4.38 (s, 2H, CH₂-Py), 2.95-2.94-2.92 (t, 2H, CH₂), 1.91-1.90-1.88-1.87-1.85 (m, 2H, CH₂).

### Synthesis of the compound L3

2,2'-[butane-1,4-diylbis (iminomethanediyl)]bis(5-hydroxy-4H-pyran-4-one) (L3). 1.0 g (7 mmol) of KA was dissolved in 20 mL of ethanol (96%) and 250 µl (3.5 mmol) of butane-1,4-diamine was added. After stirring the mixture for 1 hour at room temperature, the precipitate was filtered and washed with ethyl acetate. The identity and purity of the compound were ascertained with NMR analysis - Quantum yield 94%, melting point 168°C, ¹H NMR (500 Hz, D₂O), δ (ppm): 7.68 (s, 1H , 6-HPy), 6.36 (s, 1H, 3-HPy), 4.37 (s, 2H, CH₂-Py), 2.91 (s, 2H, CH₂), 1.63 (s, 2H, CH₂).

### Synthesis of compound L4

6,6',6"-(((nitrilotris(ethane-2,1-diyl))tris(azanediyl))tris(methylene))tris(3-hydroxy-4H-pyran-4-one) (L4). Kojic acid (1.0 g, 7.04 mmol) was suspended in 20 mL of ethanol followed by the dropwise addition of tris (2-aminoethyl)amine (350 µL, 2.32 mmol) dissolved in CHCl₃ (2 mL). After stirring the reaction mixture at room temperature for 1 hour, the precipitate was filtered and washed with ethyl acetate. The identity and purity of the sample were confirmed by NMR and elemental analysis. Analytical data: 98% quantum yield, 1H NMR (D2O, 500 MHz) δ 2.83 (t, 6H, J = 2.81 Hz), 3.09 (t, 6H, J = 3.09 Hz), 4.49 (s, 6H), 6.49 (s, 3H), 7.82 (s, 3H). 13C NMR (D2O, 500 MHz) δ 39.84, 53.86, 62.92, 112.94, 145.16, 168.24, 184.01, 199.25. Elementary analyzes (%) theoretical results (C24H36N4O12 3H2O): C 50.35; H 6.34, N 9.79%; experimental results: C 50.65, H 6.17, N 9.27%.

### Cell cultures

The commercial embryonic cell line of the human kidney 293T (Banca Biologica and Cell Factory - IRCCS AOU San Martino - IST Genova, catalog code ICLC HTL03003), that of murine macrophages J774 and RAW (ICLC ATL98011 and ATL02001), that of Glioblastoma del human brain T98G (ATCC^{®} CRL-1690^{™}) and that of human epithelial adenocarcinoma Caco-2 (ICL HTL97023), were provided by the National Institute for Research on Cancer c/o CBA (ICLC, Genoa). A minimal essential cell culture medium, MEM (EBSS), with 10% fetal bovine serum (FBS), 100 units per mL penicillin, 100 mg × mL streptomycin, 2 mM glutamine and 1% not essential amino acids was used. To reach the experimental conditions, the confluent cells were isolated using trypsin / EDTA. The cells of the different cell lines (2-3 × 10⁴ cells per cm²) were seeded in 35 mm cell discs and then incubated at 37°C in 5% CO₂. After 24 hours of growth in complete culture medium, the cells were cultured according to the different experimental conditions foreseen by the project.

The morphology of the different cell lines, normal (293T, RAW) and cancerous (T98G, CaCo2), treated with Colcemid (Sigma Aldrich) and L1 can be compared with the control samples that grow without any treatment. These morphological changes are characterized by rounded-shaped cells apparently connected to each other in linear continuity, aspects already described in the literature with respect to treatment with Colcemid. While all cell lines respond to Colcemid treatment, only the CaCo2 line and the T98G glioblastoma line treated with L1 show an arrest in M phase similar to that induced by Colcemid. The other cell lines tested, neoplastic and not, treated with L1 are apparently normal. Figure 1 shows the T98G, CaCo2 cells treated with the L1 ligand and stained with hematoxylin and eosin (Figure 1) which highlights the "thickened chromatin" (dark blue) typical of an M phase block and similar to that found with the treatment with Colcemid.

### TEM microscopy

Treated and untreated control samples of Caco-2 human epithelial adenocarcinoma cells were fixed for 2 hours in 1% paraformaldehyde (para) and 1.25% glutaraldehyde solution in 0.1 M, pH 7.4 sodium cacodylate buffer. After rinsing in the same buffer, the samples were post-fixed in 1% osmium tetroxide for 1 hour and stained in 0.25% uranyl acetate overnight at 4°C. The cell cultures were dehydrated in increasing scale of ethanol and xylene, subsequently, to proceed with the embedding in Embed 812 resin, the samples were transferred in inclusion flat molds in an oven at 60°C for the 24 hours necessary for the polymerization of the resin. From the resulting blocks, 1 µm thick sections were obtained and then stained with toluidine blue for preliminary analysis by light microscopy. Finally, thin sections of 60-90 nm (ultramicrotome 8800 LKB), contrasted with urethyl acetate and bismuth subnitrate, were observed and photographed with a transmission electron microscope (model JEOL 1400 plus, Tokyo, Japan) operating at 80 kV.

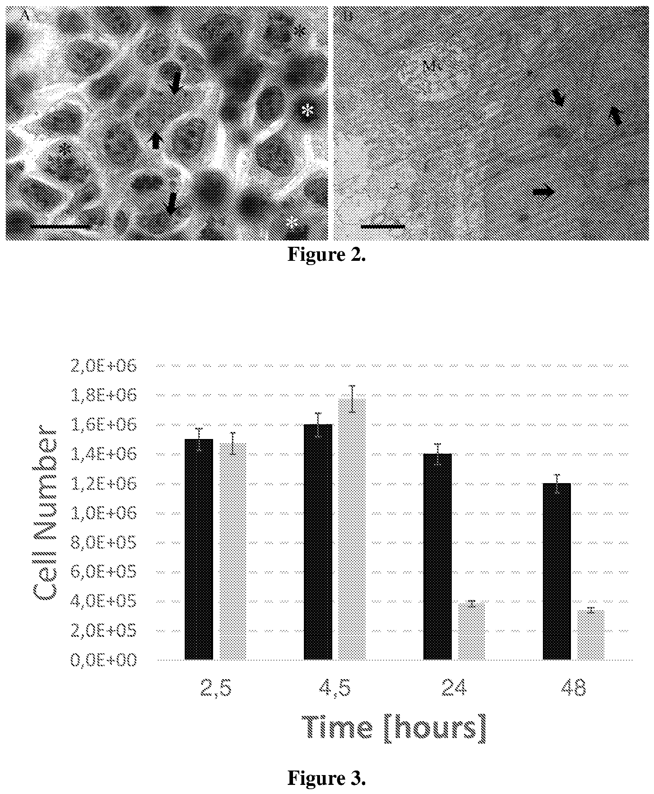

Although further studies and insights are needed on the molecular mechanisms that induce the blockade of mitosis after treatment with L1, our results clearly suggest that the compound L1 specifically inhibits the cell division process in Caco-2 cells. In these cells, compound L1 does not appear to affect the cellular architecture of most cells during the interphase (Figure 2B). However, occasionally, cells showing fragmentation of their nuclei have been observed (Fig. 2A). This singular phenomenon may be associated with the apoptotic process that could take place after L1 treatment.

### Example 2

### Cytotoxic activity: Trypan Blue staining.

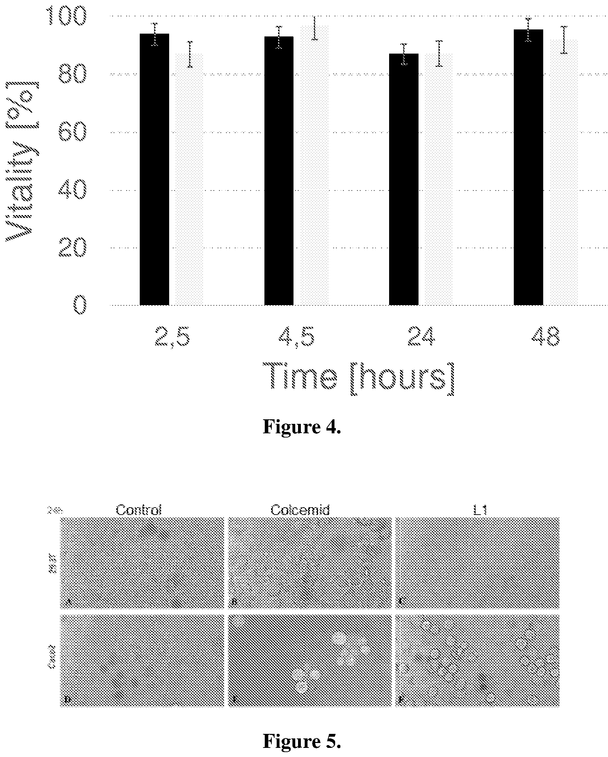

A possible cytotoxic effect (ID₅₀) of the L1 molecule was evaluated in CaCo2 cells by viable staining with trypan blue under different experimental conditions. Cell number (Fig. 3) and viability (Fig. 4) were assessed using the LunaFL cell counter (www.logosbio.com) or by direct observation under the light microscope. Briefly, the cell samples mixed and diluted 1:1 with a 0.2% Trypan Blue solution were counted, after 1-3 minutes, in the counting chamber slide present in the analyzer of the Luna automatic system. The results are reported by the system as a percentage of cell viability and number of cells relative to the untreated control samples. To obtain an accurate measurement of cell viability, the cell count was repeated at least three times in at least three experiments under the same experimental conditions. The observation under the optical microscope (Fig. 5) was made by adding the Trypan Blue solution to the cells to be observed for a few minutes. The percentage of live cells (trypan blue negative) compared to dead ones (trypan blue positive) in each experimental culture condition was then calculated.

The results obtained show a clear reduction in the number of CaCo2 cells treated for 24 hours with L1 compared to those not treated (Figure 3). The reduced number of cells does not appear to be due to cell death as the viability of these cells is comparable to that of the respective control not treated with L1. (Figure 4). Repeated treatments with L1 show a further reduction in the number of cells.

The automatic measurements made with the LunaFL system were confirmed by microscopic analysis (Figure 5). Inhibition of mitosis is evidenced by the characteristic morphological changes of blockade in mitosis (round shaped cells), but not by the blue staining with Trypan Blue.

### Example 3

### mRNA analysis

To better characterize the effect of kojic acid derivatives in cell lines 293T and CaCo2, the expression of 4 genes involved in the response to different cellular stress stimuli such as: PRDX1: this gene encodes a member of the peroxiredoxin family was evaluated at the mRNA level by real-time PCR, an antioxidant enzyme that reduces hydrogen peroxide. Coding for a protein that may play a role in the control of cell proliferation and in tumor development and progression.

BAX: it is a protein of the Bcl2 family, is involved in the foreground in cellular apoptosis, an over-expression of the RNAm of this protein indicates a propensity of the cell to self-destruction, this mechanism is regulated by the tumor suppressor P53. BAX is also associated in the evolution of colorectal cancer.

Bcl2: is the prototype of a family of genes, and of the corresponding encoded proteins, present in mammals. These proteins govern mitochondrial outer membrane permeabilization (MOMP), and can be either pro-apoptotic (Bax, BAD, Bak, Bok and others), and anti-apoptotic- (Bcl-2, Bcl-xL and Bcl-w, the main ones). The Bcl-2 protein activates apoptosis.

HO-1: it is a protein with heme-oxygenase activity in heme and non-heme substrates known above all for its anti-inflammatory activity.

The cells, after treatment, were immediately frozen and stored at -80°C until RNA extraction. The RNA was extracted with the NorDiag ARROW RNA Kit using the company's automated tool. Actin was used as a reference gene. Real-time reverse transcriptase PCR was performed using the Roche Light Cycler System and the SYBR Green I Amplification Kit (Roche Diagnostics). The primers used for the beta actin control gene are as follows: b-actF = 5'-GCATGGGTCAGAAGG-3', b-actR = 5'-AGGCGTACAGGGATAG-3', e (Gen-Bank n. NM_001101). While the following oligo nucleotides were used for the genes under consideration:
1) Gene Bax: BAX-F = 5'-GCTTCAGGGTTTCATC-3', BAX-R = 5'-CCTTGAGCACCAGTTT-3'
2) Gene Bcl2: Bcl2-F = 5'-GCCTCATGAAATAAAGAT-3', Bcl2-R = 5'-TGGATGTACTTCATCACT-3'.
3) Gene PRDX1: PRDX1-F 3'-AGGGGCCTTTTTATC-5', PRDX1-R = 3'-TGGAAAAAAAAAATACAG-5'.
4) Gene HO-1: HO1-F = 3'-TACACCCGCTACCTG-5', HO1-R = 3'-TCTGGTCCTTGGTGTC-5'.

The final reaction volume of 20 µl contained: a final concentration of 3 mM MgCl₂, 0.25 mM of each primer and 2 µl of RNA extract. The real time reaction program includes: an initial reverse transcription at 55°C for 10 minutes, a denaturation cycle at 95°C for 30 seconds followed by 35 cycles at 95°for 5 seconds, 53°C for 10 seconds and 72°C for 12 seconds, the fluorescence reading was performed in the 72°C phase

The relative gene expression was analyzed using the 2-ΔΔCT method [KJ Livak and TD Schmittgen, Analysis of relative gene expression data by Quantitative real-time PCR and 2-ΔΔCT method, methods, 2001, 25, 402-408]. Three distinct experimental replicates were performed for each analysis and the quantitative data were expressed as mean. The experiment was repeated if the dispersion of the values of the threshold cycles, compared to the mean, was too high. A standard deviation for each replicate equal to ± 1 cut-off cycle was accepted.

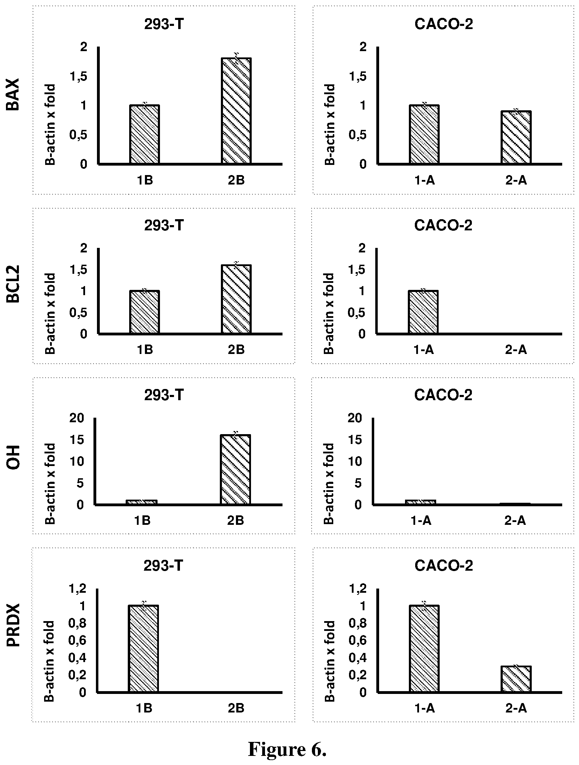

The results of this molecular analysis showed that L1 treatment in 293T cells leads to increased expression of the stress genes BAX, BCL2 and OH-1, while completely inhibiting the expression of the PRDX gene. In contrast, the L1 and cell treatment of CaCo2 significantly reduces the expression of all studied genes. (Figure 6). we can therefore conclude that, albeit in different ways, L1 treatment interferes in the expression of genes correlated with apoptosis and / or cellular stress.

## Claims

1. Compound L1 for use in therapy, with formula: 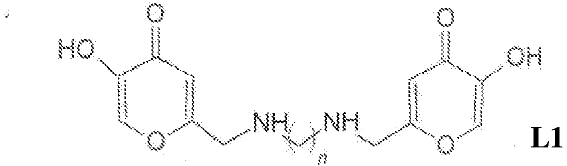 where n = 2.

2. Compound L1 for use according to claim 1 wherein the therapy is the treatment of tumors, corresponding relapses and metastases in humans and animals.

3. Compound L1 for use according to claim 2 wherein the tumor is bowel cancer, in particular colorectal cancer.

4. Compound L1 for use according to claim 2 wherein the tumor is glia tumor, in particular glioblastoma.

5. Compositions comprising the compound L1 according to claim 1 for use in therapy.

6. Compositions for use according to claim 5 wherein the therapy is the treatment of tumors, relapses and metastases in humans and animals.

7. Composition for use according to claim 6 wherein the tumor is bowel cancer, in particular colorectal cancer.

8. Composition for use according to claim 6 wherein the tumor is glia tumor, in particular glioblastoma.

9. Composition for use according to claims 5-8 which is a pediatric formulation.

10. Composition for use according to claims 5-9 further comprising at least one of: carriers, excipients and diluents such as: lactose, dextrose, sucrose, sorbitol, mannitol, xylitol, erythritol, maltitol, starch, acacia gum, alginate, gelatin, calcium phosphate, calcium silicate, cellulose, methylcellulose, polyvinylpyrrolidone, water, talc, magnesium stearate and mineral oil; fillers, anti-coagulants, lubricants, wetting agents, fragrances, emulsifiers and preservatives.

11. Composition for use according to claims 5-10 formulated to provide rapid, prolonged of delayed release of the active ingredient after administration.

12. Composition for use according to claims 5-11 in the form of powders, granules, tablets, emulsions, syrups, aerosols, soft or hard gelatin capsules, solutions, sterile injectable solutions, sterile powders.

13. Composition for use according to claims 5-12 comprising in combination a compound having an effect of prevention, improvement or treatment of tumor symptoms.

14. Composition for use according to claim 13 wherein the compound is selected from: cisplatin and its derivatives: nedaplatin, carboplatin, picolplatin, sartaplatin, lobaplatin, oxaliplatin, carboplatin; Pembrolizumab, Aflibercept, Bevacizumab, Cetuximab, Levoleucovorin, Nivolumab, Panitumumab, Tipiracil, Trifluridine, Vandetanib, 7-ethyl-10-hydroxycamptothecin, Fluorouracil, Cyanocobalamin, Capizlis-011-cycle, Nivolumabine, Capecumabicli, Capecumaconi-011 Mitomycin, Ramucirumab, Regorafenib.

15. Functional food comprising the compound L1 according to claim 1.

16. Functional food according to claim 15 formulated as food, food additive, beverage, for humans and for animals, including formulations for infant formula and baby food.

## Patentansprüche

1. Verbindung L1 zur Verwendung in der Therapie, mit der Formel 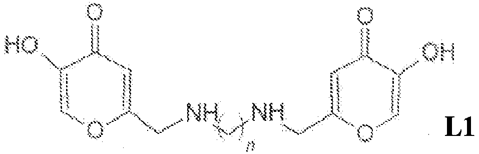 wobei n = 2.

2. Verbindung L1 zur Verwendung nach Anspruch 1, wobei die Therapie die Behandlung von Tumoren, entsprechende Rückfällen und Metastasen bei Menschen und Tieren ist.

3. Verbindung L1 zur Verwendung nach Anspruch 2, wobei der Tumor ein Darmkrebs, insbesondere ein Dickdarmtumor, ist.

4. Verbindung L1 zur Verwendung nach Anspruch 2, wobei der Tumor ein Gliatumor, insbesondere ein Glioblastom, ist.

5. Zusammensetzungen enthaltend die Verbindung L1 nach Anspruch 1 zur Verwendung in der Therapie.

6. Zusammensetzungen zur Verwendung nach Anspruch 5, wobei die Therapie die Behandlung von Tumoren, Rückfällen und Metastasen bei Menschen und Tieren ist.

7. Zusammensetzung zur Verwendung nach Anspruch 6, wobei der Tumor ein Darmkrebs, insbesondere ein Dickdarmkrebs, ist.

8. Zusammensetzung zur Verwendung nach Anspruch 6, wobei der Tumor ein Gliatumor, insbesondere ein Glioblastom, ist.

9. Zusammensetzung zur Verwendung gemäß den Ansprüchen 5-8, die eine pädiatrische Formulierung ist.

10. Zusammensetzung zur Verwendung nach Ansprüchen 5-9, weiterhin umfassend mindestens eines von: Trägern, Hilfsstoffen und Verdünnungsmitteln, wie zum Beispiel: Lactose, Dextrose, Saccharose, Sorbitol, Mannitol, Xylitol, Erythritol, Maltitol, Stärke, Gummi arabicum, Alginat, Gelatine, Calciumphosphat, Calciumsilikat, Cellulose, Methylcellulose, Polyvinylpyrrolidon, Wasser, Talk, Magnesiumstearat, Mineralöl, Füllstoff, Antikoagulanzien, Gleitmittel, Benetzungsmittel, Parfüme, Emulgatoren und/oder Konservierungsmittel.

11. Zusammensetzung zur Verwendung gemäß den Ansprüchen 5-10, die so formuliert ist, dass sie nach der Verabreichung eine schnelle, verlängerte oder verzögerte Freisetzung des Wirkstoffs ermöglicht.

12. Zusammensetzung zur Verwendung gemäß den Ansprüchen 5-11, formuliert in Form von Pulvern, Granulaten, Tabletten, Emulsionen, Sirupen, Aerosolen, Weich- oder Hartkapseln, Lösungen, sterilen injizierbaren Lösungen, sterilen Pulvern.

13. Zusammensetzung zur Verwendung gemäß den Ansprüchen 5-12, umfassend in Kombination eine Verbindung mit vorbeugender, lindernder oder heilender Wirkung bei Tumorsymptomen.

14. Zusammensetzung zur Verwendung gemäß dem Anspruch 13, wobei die Verbindung ausgewählt ist aus: Cisplatin und seinen Derivaten: Nedaplatin, Carboplatin, Picolplatin, Sartaplatin, Lobaplatin, Oxaliplatin, Carboplatin; Pembrolizumab, Aflibercept, Bevacizumab, Cetuximab, Levoleucovorin, Nivolumab, Panitumumab, Tipiracil, Trifluridin, Vandetanib, 7-Ethyl-10-hydroxycamptothecin, Fluorouracil, Cyanocobalamin, Capizlis-011-Zyklus, Nivolumabin, Capecumabicli, Capecumaconi-011 Mitomycin, Ramucirumab und Regorafenib.

15. Funktionelles Lebensmittel enthaltend die Verbindung L1 gemäß Anspruch 1.

16. Funktionelles Lebensmittel nach Anspruch 15, formuliert in Form von Nahrungsmitteln, Nahrungsmittelzusätzen oder Getränken, für Menschen und für Tiere, einschließlich Formulierungen für Säuglings- und Säuglingsnahrung.

## Revendications

1. Composé L1 destiné à être utilisé dans une thérapie, avec la formule 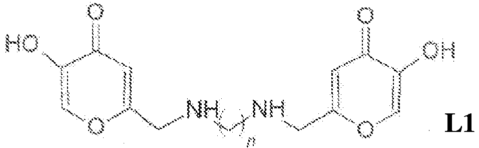 dans lequel n = 2.

2. Composé L1 destiné à être utilisé selon la revendication 1, dans lequel la thérapie est le traitement des tumeurs, des correspondantes relapses et métastases dans les personnes et les animaux.

3. Composé L1 destiné à être utilisé selon la revendication 2, dans lequel la tumeur est un cancer de l'intestin, en particulier un cancer colorectal.

4. Composé L1 destiné à être utilisé selon la revendication 2, dans lequel la tumeur est une tumeur gliale, en particulier un glioblastome.

5. Compositions comprenant le composé L1 selon la revendication 1, pour une utilisation en thérapie.

6. Compositions destinées à être utilisées selon la revendication 5, dans lesquelles la thérapie est le traitement des tumeurs, relapses et métastases dans les humains et les animaux.

7. Composition destinée à être utilisée selon la revendication 6, dans laquelle la tumeur est un cancer à l'intestin, en particulier un cancer colorectal.

8. Composition destinée à être utilisée selon la revendication 6, dans laquelle la tumeur est une tumeur gliale, en particulier un glioblastome.

9. Composition destinée à être utilisée selon les revendications 5-8, qui est une formulation pédiatrique.

10. Composition destinée à être utilisée selon les revendications 5-9, comprenant en outre au moins l'un parmi : des supports, des excipients et des diluants, tels que: lactose, dextrose, saccharose, sorbitol, mannitol, xylitol, érythritol, maltitol, amidon, gomme arabique, alginate, gélatine, phosphate de calcium, silicate de calcium, cellulose, méthylcellulose, polyvinylpyrrolidone, eau, talc, stéarate de magnésium, huile minérale, une charge, des anticoagulants, des lubrifiants, des agents mouillants, des parfums, des émulsifiants et/ou des conservateurs.

11. Composition destinée à être utilisée selon les revendications 5-10, formulé pour fournir une libération rapide, prolongée ou retardée de l'ingrédient actif après administration.

12. Composition destinée à être utilisée selon les revendications 5-11, formulée sous forme de poudres, granulés, comprimés, émulsions, sirops, aérosols, gélules molles ou dures, solutions, solutions injectables stériles, poudres stériles.

13. Composition destinée à être utilisée selon les revendications 5-12, comprenant en combinaison un composé ayant un effet de prévention, d'amélioration ou de traitement des symptômes tumoraux.

14. Composition destinée à être utilisée selon la revendication 13, dans lequel le composé est choisi entre: cisplatine et ses dérivés: nédaplatine, carboplatine, picolplatine, sartaplatine, lobaplatine, oxaliplatine, carboplatine; Pembrolizumab, Aflibercept, Bevacizumab, Cetuximab, Levoleucovorin, Nivolumab, Panitumumab, Tipiracil, Trifluridine, Vandetanib, 7-éthyl-10-hydroxycamptothécine, Fluorouracile, Cyanocobalamine, Capizlis-011-cycle, Nivolumabine, Capecumabicli, Capecumaconi-011 Mitomycine, Ramucirumab et Régorafénib.

15. Aliment fonctionnel comprenant le composé L1 selon la revendication 1.

16. Aliment fonctionnel selon la revendication 15, formulé sous forme d'aliment, d'additif alimentaire ou de boisson, pour les humains et pour les animaux, comprenant des formulations pour de formules d'alimentation des infants et des bébés.
